# EUROPEAN PATENT SPECIFICATION

(11) **EP 2 079 446 B1**
(45) Date of publication and mention of the grant of the patent: **26.10.2011**
(21) Application number: 08795541.5
(22) Date of filing: 21.08.2008
(51) Int. Cl.: A61K 9/16, A61K 9/20, A61K 9/50

(54) **PALIPERIDONE SUSTAINED RELEASE FORMULATION**
PALIPERIDONFORMULIERUNG MIT VERZÖGERTER FREISETZUNG
FORMULATION À LIBÉRATION PROLONGÉE DE PALIPÉRIDONE

(30) Priority: 21.08.2007 US 935597 P
(43) Date of publication of application: 22.07.2009
(73) Proprietor: Teva Pharmaceutical Industries Ltd., 49131 Petah Tiqva (IL)
(72) Inventor: FOX, Michael, 67291 Tel Aviv (IL); DI CAPUA, Simona, 44627 Kfar Saba (IL); REINBERG, Ronny, Modii'n (IL)
(74) Representative: Russell, Tim
(86) International application number: PCT/US2008/010014
(87) International publication number: WO 2009/025859

(56) References cited:
- WO-A-2004/010981
- WO-A-2004/108067
- US-A1- 2005 232 990
- US-A1- 2006 034 927
- US-A1- 2006 189 635

## Description

CROSS REFERENCE TO RELATED APPLICATION

The present application claims the benefit of U.S. Provisional Application No. 60/935,597, filed August 21, 2007.

The present invention relates to sustained release pharmaceutical compositions comprising Paliperidone or a salt thereof, and a process for preparing the same.

### BACKGROUND OF THE INVENTION

Paliperidone is described in U.S. Pat. No. 4,804,663. The paliperidone compound differs from risperidone and related prior art compounds described in U.S. Pat. Nos. 4,352,811 and 4,458,076 by its substitution on the 1-position of the piperidine moiety.

Paliperidone (CAS Registry No. 144598-75-4) has the chemical name 4H-Pyrido[1,2-a]pyrimidin-4-one, 3-[2-[4-(6-fluoro-1,2-benzisoxazol-3-yl)-1-piperidinyl]ethyl]-6,7,8,9-tetrahydro-9-hydroxy-2-methyl. Paliperidone is represented by the structural formula:

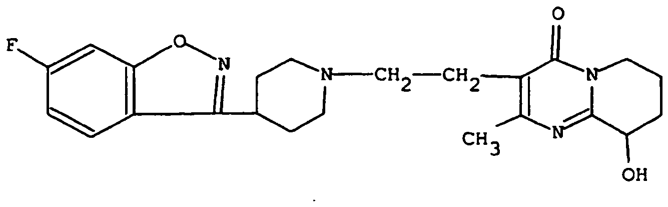

Paliperidone is practically insoluble in water, freely soluble in methylene chloride and soluble in methanol and 0.1 N hydrochloric acid. Additionally, since paliperidone has a long half-life of about one day, it is not a typical candidate for extended delivery. However, side effects such as anxiety, somnolence, dizziness, constipation, extrapyramidal symptoms, may be related to high blood plasma concentration levels restricting the ability to administer a single daily immediate release dose.

A published patent application, US 2004/0092534, discloses extended release formulations and methods for providing ascending rate of release of paliperidone utilizing a capsule-shaped tablet. The dosage form utilizes a semipermiable membrane surrounding a three layer core: the first layer contains low amounts of drug and an osmotic agent; the middle layer contains higher amounts of drug and without osmotic agent and the third layer is a push layer. In addition to the said structure of capsules shape tablet, there is at least one orifice which drilled through the membrane on the first drug layer end. All this capsule shaped tablet is designed to be a once-a day dosage form.

U.S. patent application publication No. US 2006/034927 relates also to a Paliperidone dosage form for sustained release of a drug comprising: a delay layer comprising (i) a polymeric matrix, and (ii) microencapsulated drug, wherein the delay layer is substantially free of non-microencapsulated drug; and a second layer comprising (iii) a polymeric matrix, and (iv) non-microencapsulated drug matrix; wherein the second layer is located adjacent to the delay layer.

U.S. patent application publication No. US2006/189635 relates to sustained release dosage forms of benzisoxazole derivatives.

U.S. patent application publication No. US2005/232990 relates to sustained release dosage forms of donepezil, optionally in combination with antipsychotics.

WO2004/108067 relates to programmed drug delivery systems consisting of a core and a coating.

WO2004/010981 relates to dosage forms for providing a controlled release of paliperidone.

The difficulties with the above mentioned dosage forms are being of low cost effectiveness, requiring very special and expensive equipment and resulting in relatively small production of final dosage form.

Accordingly, there remains a need to provide alternative means of controlling delivery.

### SUMMARY OF THE INVENTION

The present invention provides an extended release tablet of Paliperidone in the form of an inlay tablet. The inlay tablet comprises at least an inlay core and outer layer: (a) the inlay core comprising non-coated Paliperidone and at least one polymer capable of delaying the release of Paliperidone from the inlay core and capable of swelling upon hydration, wherein the inlay core optionally further comprises coated Paliperidone; and (b) the outer layer comprising a pharmaceutical excipient which is substantially water insoluble, wherein the outer layer partially surrounds the inlay core.

The invention provides a dosage form for the sustained release of Paliperidone, wherein the dosage form exhibits relative bioavailability, based on the area under the plasma concentration curve (AUC) for the same duration after oral administration in human subjects, of between about 1.5 and about 3.0, preferably between about 1.7 and about 3.0, and more preferably between about 1.9 and about 3.0, compared with commercially available INVEGA® extended release tablets containing the same amount of Paliperidone administered at the same dose in the human subjects.

The invention also provides a dosage form for the sustained release of Paliperidone, wherein the dosage form exhibits a relative Cmax, based on the plasma concentrations at various time after oral administration in human subjects, of between about 1.6 and about 3.0, preferably between about 1.7 and about 3.0, and more preferably between about 2.0 and about 3.0, compared with commercially available INVEGA® extended release tablets containing the same amount of Paliperidone, administered at the same dose in the human subjects.

In another aspect, the invention provides a process of making the inlay tablet of the invention, wherein the process comprises
(1) mixing Paliperidone and at least one polymer capable of delaying the release of Paliperidone and capable of swelling upon hydration, wherein at least part of the Paliperidone remains non-coated; and
(2) partially covering the mixture of step (1) with an outer layer comprising a pharmaceutical excipient which is substantially water insoluble to obtain the inlay tablet.

### BRIEF DESCRIPTION OF THE DRAWINGS

Figure 1 is a schematic diagram showing an embodiment of the inlay tablet of the present invention, comprising an inlay core containing non-coated Paliperidone partially surrounded or incompletely covered by an inert insoluble outer layer.
Figure 2 shows the dissolution profiles of the Paliperidone extended release tablets in the form of inlay tablets prepared according to Example 8.
Figure 3 shows the least-square mean plasma concentrations versus time after the oral administration of the 6 mg inlay tablets of the present invention in the Test Group and the oral administration of the INVEGA 6 mg commercially available Paliperidone tablets in the Reference Group.
Figure 4 shows the natural logarithm of the least-square mean plasma concentrations versus time after the oral administration of the 6 mg inlay tablets of the present invention in the Test Group and the oral administration of the INVEGA 6 mg commercially available Paliperidone tablets in the Reference Group.

### DETAILED DESCRIPTION OF THE INVENTION

In the present invention, the term "coated Paliperidone" means one or more Paliperidone particles which have been microencapsulated with at least one microencapsulation material. Such materials include, but are not limited to, proteins, polysaccharides, starches, waxes, fats, natural and synthetic polymers, and resins and/or combinations thereof. In this invention, "non-coated Paliperidone" means one or more Paliperidone particles which have not been microencapsulated with any microencapsulation material.

The term "microencapsulated with at least one microencapsulating material" means that the Paliperidone particles are surrounded by a layer of the at least one microencapsulating material without any pharmaceutical excipients existing between the Paliperidone particles and the layer of the at least one microencapsulating material.

In the present invention, the term "polymer" comprises natural and/or synthetic polymers and can also mean a combination of polymers of various types. The "polymer having an effect of delaying Paliperidone release" or "polymer capable of delaying the release of Paliperidone" includes polymers that form a viscous and gelatinous surface barrier or gel layer upon hydration, which barrier or gel layer controls Paliperidone release from and the penetration of liquids into the center of Paliperidone particles. The physicochemical characteristics of this barrier or gel layer control water uptake and the mechanism of Paliperidone release from the Paliperidone particles. The release of Paliperidone can occur via diffusion of Paliperidone through the barrier or gel layer, and preferably via gradual erosion of the barrier or gel layer. Suitable examples of the "polymer having an effect of delaying Paliperidone release" or "polymer capable of delaying the release of Paliperidone" include polyvinylpyrrolidone, polyethylene oxide such as POLYOX WSR-301, polysaccharides and hydrophilic cellulose derivatives such as methyl cellulose, hydroxypropyl methylcellulose, hydroxyethyl cellulose, hydroxypropyl cellulose, hydroxyethyl methylcellulose, carboxy methylcellulose and sodium carboxy methylcellulose. Preferred examples of the "polymer having an effect of delaying Paliperidone release" or "polymer capable of delaying the release of Paliperidone" include polyethylene oxide and hydroxypropyl methylcellulose such as METHOCEL K15MP, K15M, K100M, K100LV, F4M, E4M, E3, E5, E10M, E15LV, E15LN, E15CLV, E50 and K3.

The term "delay layer" means a layer that functions, at least in part, to retard the release of the drug from the dosage form, including halting the release for a certain period of time.

In the invention, when two components (or two layers) are "adjacent", that means the two components (or two layers) are in physical proximity with each other. Preferably, the two components (or two layers) are in direct contact at least one point.

In the invention, the term "core" refers to a component that is at least partially surrounded or covered by another component.

As used herein, the term "ascending release kinetics" means that the amount of Paliperidone released as a function of time increases over a period of time. Preferably, the amount of Paliperidone released as a function of time increases continuously, gradually and/or steadily, instead of in a step-wise fashion.

The term "sustained release dosage form" means a dosage form that releases the drug for 4-24 hours. The dosage forms exhibit t90 values of at least 4 hours or more and preferably up to about 24 hours or more, for once per daily dosing. The dosage forms continuously release drug for sustained periods of at least about 6 hours, preferably about 8 hours or more and, in particular embodiments, about 12 hours or more.

The invention is directed to Paliperidone extended release tablets in the form of inlay tablets. The inlay tablet comprises at least an inlay core and outer layer: (a) the inlay core comprising non-coated Paliperidone and at least one polymer capable of delaying the release of Paliperidone from the inlay core and capable of swelling upon hydration, wherein the inlay core optionally can further comprise coated Paliperidone; and (b) the outer layer comprising a pharmaceutical excipient which is substantially water insoluble, wherein the outer layer partially surrounds the inlay core.

The at least one "polymer capable of delaying the release of Paliperidone from the inlay core and capable of swelling upon hydration" is at least one polymer that forms a viscous and gelatinous surface barrier or gel layer upon hydration, which barrier or gel layer controls Paliperidone release from and the penetration of liquids into Paliperidone particles. The release of Paliperidone can occur via diffusion of Paliperidone through the barrier or gel layer, and/or via gradual erosion of the barrier or gel layer. Suitable examples of the polymer include polyvinylpyrrolidone, poly(ethylene oxide) such as POLYOX WSR-301, polysaccharides and hydrophilic cellulose derivatives such as methyl cellulose, ethyl cellulose, hydroxypropyl methylcellulose, hydroxyethyl cellulose, hydroxypropyl cellulose, hydroxyethyl methylcellulose, carboxy methylcellulose and sodium carboxy methylcellulose. Preferred examples of the "polymer having an effect of delaying Paliperidone release from the particles" include poly(ethylene) oxide and hydroxypropyl methylcellulose such as METHOCEL K15MP, K15M, K100M, K100LV, F4M, E4M, E3, E5, E15M, E15LV, E15LN, E15CLV, E50 and K3. A preferred at least one "polymer capable of delaying the release of Paliperidone from the inlay core and capable of swelling upon hydration" is poly(ethylene) oxide.

The "pharmaceutical excipient which is substantially water insoluble" of the outer layer renders the outer layer substantially water insoluble. Examples of "pharmaceutical excipient which is substantially water insoluble" in the outer layer include pharmaceutically acceptable polymers which are substantially water insoluble, such as pharmaceutically acceptable drug release modifying polymers which are substantially water insoluble. Suitable pharmaceutically acceptable polymers which are substantially water insoluble include cationic copolymers of ethylacrylate and methylacrylate with quarternary ammonium groups such as EUDRAGIT RS and EUDRAGIT RL, ethylacrylate methylmethacrylate copolymer with neutral ester groups, cellulose esters, cellulose ethers and cellulose esterethers, ethyl cellulose such as ETHYL CELLULOSE T10 PHARM, cellulose acetate, cellulose diacetate, cellulose triacetate and polyester polymers. Non-limiting examples of the polyester polymers that can be used include poly(ε-caprolactone)s, poly(alkylene glycol adipate)s such as poly(ethylene glycol adipate), poly(propylene glycol adipate) and poly(butylene glycol adipate), polyvinyl acetate and blends and copolymers thereof. A preferred "pharmaceutical excipient which is substantially water insoluble" is ethyl cellulose.

The inlay core preferably is in the form of a tablet or compressed slug.

The inlay core and the outer layer independently can further comprise at least one other pharmaceutical excipient such as pharmaceutically acceptable fillers, diluents, pH modifiers, glidants, lubricants, binders, dyes and flavoring agents.

In some of the embodiments of the inlay tablet of the invention, the inlay core comprises about 1-3% w/w Paliperidone, about 2-5% w/w filler such as STARLAC, about 5-15% w/w pH modifier such as magnesium carbonate, about 5-20% w/w release modifying polymer such as poly(ethylene) oxide, about 0-1% w/w lubricant such as stearic acid and about 0-1% w/w glidant such as silicon dioxide.

In some of the embodiments of the inlay tablet of the invention, the outer layer comprises about 50-90% w/w release modifying polymer such as ethyl cellulose, about 0-1% w/w dye such as FERRIC OXIDE YELLOW NF and about 0-1% w/w lubricant such as stearic acid.

The invention provides a dosage form for the sustained release of Paliperidone, wherein the dosage form exhibits relative bioavailability, based on the area under the plasma concentration curve (AUC) for the same duration, e.g., 0 to 96 hours, or 0 hour to infinity, after oral administration, of at least about 1.5, preferably at least about 1.7, and more preferably at least about 1.9, compared with commercially available INVEGA® extended release tablets containing the same amount of Paliperidone administered at the same dose. By a relative bioavailability, based on AUC, of at least about 1.5, it means that the AUC achieved in the human subjects orally administered the dosage form according to the first aspect of the invention is at least about 50% higher than the AUC, for the same duration, achieved in the human subjects orally administered the commercially available INVEGA® extended release tablets containing the same amount of Paliperidone, wherein the Paliperidone is administered at the same dose.

The invention also provides a dosage form for the sustained release of Paliperidone, wherein the dosage form exhibits relative bioavailability, based on the area under the plasma concentration curve (AUC) for the same duration after oral administration, of between about 1.5 and about 3.0, preferably between about 1.7 and about 3.0, and more preferably between about 1.9 and about 3.0, compared with commercially available INVEGA® extended release tablets containing the same amount of Paliperidone administered at the same dose.

Another aspect of the invention is directed to a dosage form for the sustained release of Paliperidone, wherein the dosage form exhibits a relative Cmax, based on the plasma concentrations at various time after oral administration in human subjects, of at least about 1.6, preferably at least about 1.7, and more preferably at least about 2.0, compared with commercially available INVEGA® extended release tablets containing the same amount of Paliperidone, administered at the same dose in the human subjects.

The invention also provides a dosage form for the sustained release of Paliperidone, wherein the dosage form exhibits a relative Cmax, based on the plasma concentrations at various time after oral administration in human subjects, of between about 1.6 and about 3.0, preferably between about 1.7 and about 3.0, and more preferably between about 2.0 and about 3.0, compared with commercially available INVEGA® extended release tablets containing the same amount of Paliperidone, administered at the same dose in the human subjects.

The invention provides a dosage form for the sustained release of Paliperidone, wherein the dosage form exhibits an in vitro dissolution profile determined using a 50 RPM paddle method in a dissolution medium of 500 ml 0.05 M phosphate buffer, pH 6.8, 37oC, wherein the dissolution profile is less than about 10% dissolution in 4 hours, between about 10% to about 25% dissolution in 8 hours, between about 40% to about 60% dissolution in 16 hours and not less than about 70% in 24 hours after the start of the dissolution study, respectively.

In a further aspect, the invention provides a process of making the inlay tablet of the invention, wherein the process comprises
(1) mixing Paliperidone and at least one polymer capable of delaying the release of Paliperidone and capable of swelling upon hydration; and
(2) partially covering the mixture of step (1) with an outer layer comprising a pharmaceutical excipient which is substantially water insoluble to obtain the inlay tablet.

In a preferred embodiment of the process of preparing the inlay tablet, the mixture of step (1) is compressed into a slug or tablet before step (2).

In a further preferred embodiment of the process of preparing the inlay tablet, the mixture of step (1) is compressed into a slug or tablet before step (2); the slug or tablet is mixed with the at least one polymer capable of delaying the release of Paliperidone and capable of swelling upon hydration to form a mixture; and the mixture is compressed into a tablet before step (2). More preferably, a filler, pH modifier, glidant and/or lubricant is added in step (1). Optionally, the slug or tablet formed by compression is milled before being mixed with the at least one polymer capable of delaying the release of Paliperidone and capable of swelling upon hydration to form the mixture to be compressed into a tablet before step (2).

Preferably, in the process of preparing the inlay tablet, the product of step (2) is compressed to obtain the inlay tablet.

Some of the embodiments of the inlay tablets such as exemplified in Example 1 can be prepared according to the ingredients listed in the table below.

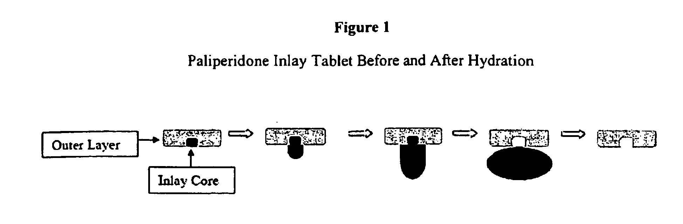

Without being bound to any hypothesis, it is believed that the inlay tablet may function in the extended release of Paliperidone according to a theory schematically illustrated in Fig. 1, which shows an embodiment of the inlay tablet of the present invention. The tablet drawn in the far left of Fig. 1 depicts the inlay tablet before exposure to an aqueous medium, and the four tablets drawn in the right depict four stages of the inlay tablet exposed to an aqueous medium for increasing length of time demonstrating gel formation in the inlay core and the gradual swelling of the gel of the inlay core due to the absorption of water by the inlay core through the surface not covered by the inert insoluble outer layer, resulting in a gradual increase of the free surface of the inlay core leading to an ascending release of Paliperidone from the inlay core.

In all aspects of the invention described herein, the pharmaceutical composition preferably further comprises one or more pharmaceutical excipients.

The term "pharmaceutical excipients" means any pharmaceutically acceptable substances, other than the active drug substance or fmished dosage form, that have been appropriately evaluated for safety and are included in drug delivery systems to (a) help in the processing of the drug delivery system during its manufacture; (b) support, protect or enhance the stability and/or bioavailability of the active drug substance; (c) make the active drug substance or the final dosage form more acceptable by the patients; (d) enhance the overall safety, effectiveness or delivery of the active drug substance during storage or use; or (e) help in product identification. The pharmaceutical excipients are added to aid the formulation and manufacture of the final dosage form for administration to the patients. The pharmaceutical excipients can be mixed with the active drug substance to make the final dosage form. Examples of "pharmaceutical excipients" include pharmaceutical grade fillers, diluents, pH modifiers, release modifying polymers, lubricants, glidants, disintegrants, carriers, bulking agents, binders, wetting agents, dyes (e.g., ferric oxide yellow and iron oxide red) and flavoring agents. Other excipients that may be incorporated into the final dosage form include preservatives, surfactants, antioxidants and any other excipient commonly used in the pharmaceutical industry.

Suitable fillers and diluents include, but are not limited to, cellulose-derived materials like powdered cellulose, microcrystalline cellulose (e.g. Avicel®), microfine cellulose, methyl cellulose, ethyl cellulose, hydroxyethyl cellulose, hydroxypropyl cellulose (e.g. Klucel®), hydroxypropyl methylcellulose, carboxymethyl cellulose salts (such as carboxymethyl cellulose calcium) and other substituted and unsubstituted celluloses; starch such as maize starch; pregelatinized starch; lactose, preferably lactose monohydrate (e.g. Pharmatose®); talc; waxes; sugars; sugar alcohols like mannitol and sorbitol; acrylate polymers and copolymers; dextrates; dextrin; dextrose; maltodextrin; pectin; gelatin; inorganic diluents like calcium carbonate, dibasic calcium phosphate dihydrate, tribasic calcium phosphate, calcium sulfate, magnesium carbonate, magnesium oxide, sodium chloride, combine materials like STARLAC and other diluents known to the pharmaceutical industry. More preferred fillers include talc, lactose monohydrate, pregelatinized starch, mannitol or sorbitol. An even more preferred filler is STARLAC.

Suitable pH modifiers are pharmaceutically acceptable buffering compounds such as alkaline earth metal carbonates, alkali metal carbonates, alkaline earth metal bicarbonates, alkali metal bicarbonates and magnesium oxide, e.g., magnesium carbonate, calcium carbonate, magnesium bicarbonate, calcium bicarbonate, sodium carbonate, potassium carbonate, sodium bicarbonate and potassium bicarbonate. A preferred pH modifier is magnesium carbonate.

Suitable disintegrants include croscarmellose sodium (e.g. Ac Di Sol®, Primellose®), crospovidone (e.g. Kollidon®, Polyplasdone®), microcrystalline cellulose, polacrilin potassium, powdered cellulose, pregelatinized starch, sodium starch glycolate (e.g. Explotab®, Primoljel®) and starch. Preferred disintegrants include Copovidone and microcrystalline cellulose.

Glidants can be added to improve the flowability of a solid composition before compaction and to improve the accuracy of dosing especially during compaction and capsule filling. Excipients that may function as glidants include colloidal silicon dioxide, magnesium trisilicate, powdered cellulose, and talc. The preferred glidant is colloidal silicon dioxide.

A lubricant may be added to the pharmaceutical compositions of the present invention to reduce adhesion and/or ease the release of the product from e.g. the die. Suitable lubricants include, but are not limited to, stearic acid, magnesium stearate, calcium stearate, glyceryl monostearate, glyceryl palmitostearate, hydrogenated castor oil, hydrogenated vegetable oil, mineral oil, polyethylene glycol, sodium lauryl sulfate, sodium stearyl fumarate, stearic acid, talc and zinc stearate. Stearic acid and magnesium stearate are preferred.

Carriers include, but are not limited to, lactose, white sugar, sodium chloride, glucose, urea, starch, calcium carbonate, kaolin, crystalline cellulose, and silicic acid.

Binders include, but are not limited to, carboxymethyl cellulose, shelac, methyl cellulose, hydroxypropyl methylcellulose, HPMC, starch and polyvinylpyrrolidone. Other suitable binders include, but are not limited to, acacia gum, pregelatinized starch, sodium alginate, glucose and other binders used in wet and dry granulation and direct compression tableting processes.

Flavoring agents and flavor enhancers make the dosage form more palatable to the patient. Common flavoring agents and flavor enhancers for pharmaceutical products that can be included in the composition of the present invention include, but are not limited to, maltol, vanillin, ethyl vanillin, menthol, citric acid, fumaric acid, ethyl maltol, and tartaric acid.

According to the preferred embodiment, the total amount of Paliperidone in the dosage form ranges from about 1 mg to about 15 mg.

Some of the embodiments of the dosage form of the invention do not contain coated Paliperidone.

Some of the embodiments of the dosage form of the invention may contain coated Paliperidone.

In some of the embodiments of the dosage form of the invention, the dosage form may contain coated Paliperidone along with non-coated Paliperidone in one or more of the components.

Methods for making multilayered are described by W. C. Gunsel, Compression coated and layer tablets in Pharmaceutical Dosage Forms: Tablets, Vol 1, edited by H.H. Lieberman.

### Examples

### Example 1 (Paliperidone extended release tablets in the form of inlay tablets)

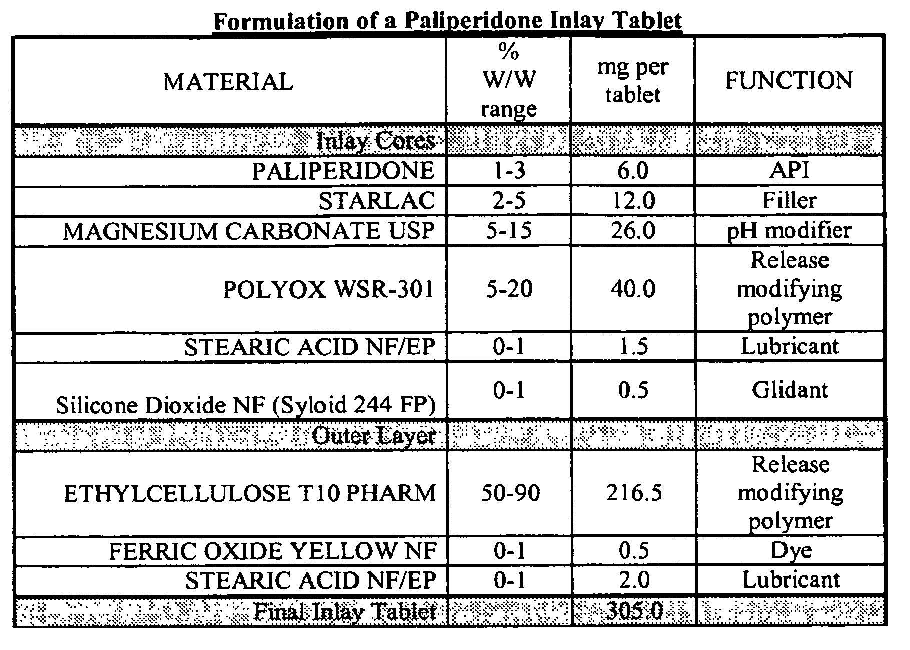

Paliperidone extended release tablets in the form of inlay tablets were prepared with the following process.

### Process

1. 90 g Paliperidone, 180 g STARLAC, 390 g magnesium carbonate, 75 g POLYOX WSR-301 and 7.5 g silicone dioxide were sieved and mixed in a V-blender.
2. 15 g Stearic acid was sieved and added to the blend from step 1 and mixed.
3. The blend from step 2 was then compressed into slugs using a 20 mm flat punch.
4. The slugs were milled using a 0.8 mm screen.
5. 378.75 g of Milled slugs were then mixed with 272.5 g POLYOX WSR-301 and 3.75 g sieved stearic acid in a V-blender.
6. The blend from step 5 was then compressed into 86 mg tablets using a 5.5 mm normal concave punch, wherein the 86 mg tablets were used as the inlay cores in the rest of the process.
7. 3247.5 g of ETHYLCELLULOSE T10 PHARM and 7.5 g yellow ferric oxide were sieved and mixed in a V-blender.
8. 30 g Stearic acid was sieved and added to the blend from step 7 and mixed.
9. The tablets from step 6 were then recompressed with the blend from step 8 to create an incomplete outer layer on the tablets from step 6 in order to form inlay tablets, each weighing 305 mg, as the Paliperidone extended release tablets, wherein each of the tablets from step 6 acts as the inlay core for the Paliperidone extended release tablets.

### Example 2 (Dissolution profile of the inlay tablets)

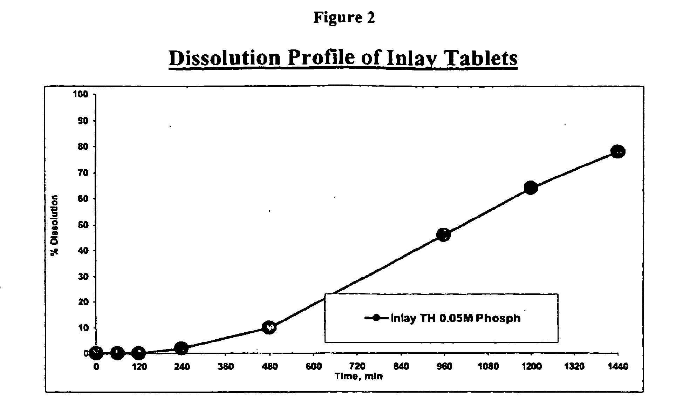

The dissolution of Paliperidone in the Paliperidone extended release tablets prepared as described in Example 8 were determined using a 50 RPM paddle method in a dissolution medium of 500 ml 0.05 M phosphate buffer, pH 6.8, wherein the dissolution was measured from 0 to 1440 minutes, i.e., 0 to 24 hours. The dissolution data are shown in Fig. 2.

### Example 3 (Pharmacokinetics of the inlay tablets)

The pharmacokinetics of the Paliperidone extended release tablets, in the form of the inlay tablets containing 6 mg Paliperidone, of the present invention were determined in a group of 16 human subjects orally administered the inlay tablets. For comparison purposes, the pharmacokinetics of commercially available Paliperidone tablets, INVEGA 6 mg, were also determined in the 16 human subjects after oral administration. Plasma concentrations of Paliperidone in the 16 human subjects were measured at 0 to 96 hours after oral administration of the Paliperidone extended release tablets of the present invention (i.e., the Test Group) or after oral administration of the commercially available INVEGA 6 mg Paliperidone tablets (i.e., the Reference Group).

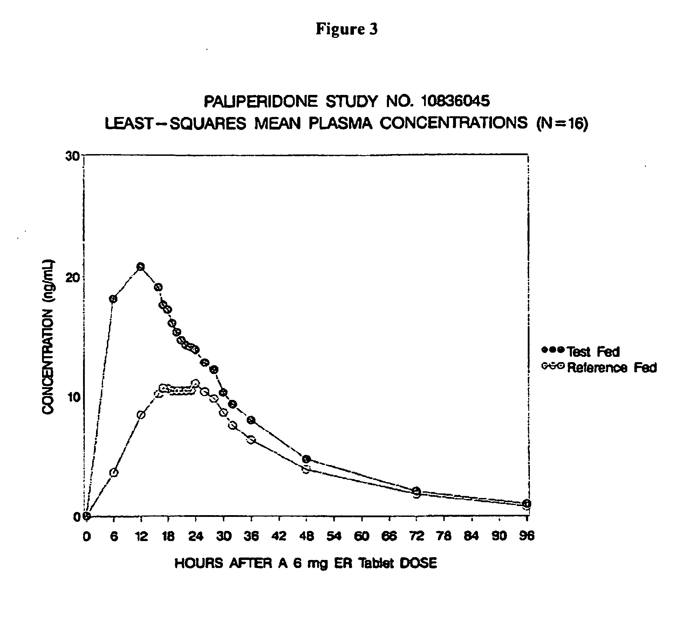

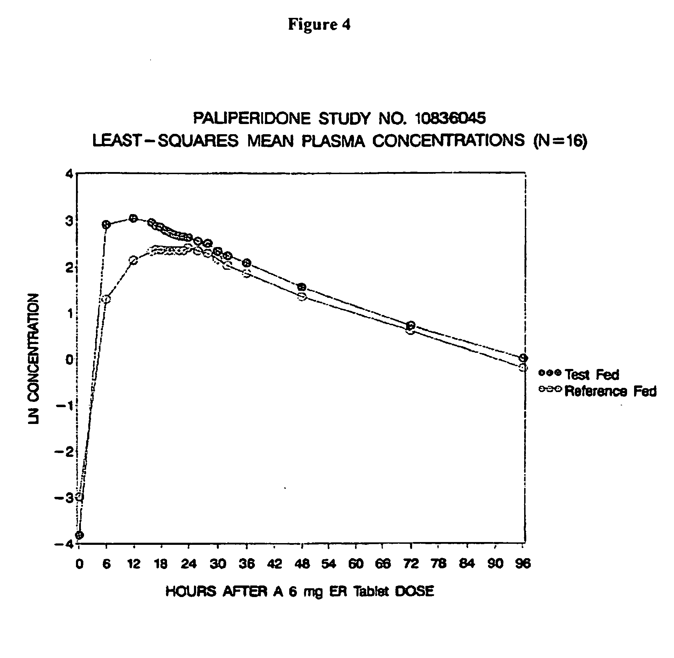

The least-square mean plasma concentrations versus time after the oral administration of the Test Group and Reference Group are shown in Figure 3. The natural log of the least-square mean plasma concentrations versus time after the oral administration of the Test Group and Reference Group are shown in Figure 4. The mean values of the pharmacokinetic (PK) parameters of the Test Group and Reference Group are shown in the table below.

### Mean PK Parameter Values

| PK Parameters | Test Group | Reference Group |
|---|---|---|
| AUC₉₆ₕ (h.ng/ml) | 702.13 | 435.94 |
| AUC_{0-∞} (h.ng/ml) | 733.43 | 461.10 |
| Cₘₐₓ (ng/ml) | 22.33 | 12.82 |
| T_{1/2} (h) | 20.17 | 19.96 |
| kₑ | 0.04 | 0.04 |
| LN AUC₉₆ₕ | 632.38 | 357.42 |
| LN AUC_{0-∞} | 659.11 | 375.70 |
| LN Cₘₐₓ | 20.33 | 10.59 |

The ratio of the mean values of the pharmacokinetic (PK) parameters of the Test Group and Reference Group, as well as the 90% confidence intervals (CI) are shown in the table below.

| Ratios of Mean PK Parameter Values (Test Group/Reference Group) Treatment | | | | |
|---|---|---|---|---|
| PK Parameters | Ratio | Lower CI | Higher CI | Effect (p) |
| AUC₉₆ₕ | 1.6106 | 1.2223 | 1.9990 | 0.0151 |
| AUC_{0-∞} | 1.5906 | 1.2012 | 1.9800 | 0.0182 |
| Cₘₐₓ | 1.7416 | 1.3451 | 2.1381 | 0.0053 |
| T_{1/2} | 1.0109 | 0.9617 | 1.0600 | |
| kₑ | 1.0041 | 0.9606 | 1.0477 | |
| LN AUC₉₆ₕ | 1.7693 | 1.4459 | 2.1651 | 0.0002 |
| LN AUC_{0-∞} | 1.7544 | 1.4356 | 2.1439 | 0.0002 |
| LN Cₘₐₓ | 1.9192 | 1.5686 | 2.3481 | 0.0001 |

## Claims

1. Paliperidone extended release tablet in the form of an inlay tablet comprising:
(a) an inlay core comprising non-coated Paliperidone and at least one polymer capable of delaying the release of Paliperidone from the inlay core and capable of swelling upon hydration; and
(b) an outer layer comprising a pharmaceutical excipient which is substantially water insoluble, wherein the outer layer partially surrounds the inlay core.

2. The extended release tablet of claim 1, wherein the at least one polymer of the inlay core is selected from the group consisting of polyvinylpyrrolidone, poly(ethylene oxide), polysaccharides, hydrophilic cellulose derivatives, methyl cellulose, ethyl cellulose, hydroxypropyl methylcellulose, hydroxyethyl cellulose, hydroxypropyl cellulose, hydroxyethyl methylcellulose, carboxy methylcellulose and sodium carboxy methylcellulose, preferably the at least one polymer of the inlay core is selected from the group consisting of methyl cellulose, ethyl cellulose, hydroxypropyl methylcellulose, hydroxyethyl cellulose, hydroxypropyl cellulose, hydroxyethyl methylcellulose, carboxy methylcellulose and sodium carboxy methylcellulose, more preferably the at least one polymer of the inlay core is selected from the group consisting of poly(ethylene oxide) and hydroxypropyl methylcellulose.

3. The extended release tablet of claim 1 or claim 2, wherein the pharmaceutical excipient which is substantially water insoluble of the outer layer is a pharmaceutically acceptable polymer which is substantially water insoluble, preferably said pharmaceutically acceptable polymer which is substantially water insoluble is selected from the group consisting of cationic copolymers of ethylacrylate and methylacrylate with quarternary ammonium groups, ethylacrylate methylmethacrylate copolymer with neutral ester groups, cellulose esters, cellulose ethers and cellulose esterethers, ethyl cellulose such as cellulose acetate, cellulose diacetate, cellulose triacetate and polyester polymers, poly(ε-caprolactone)s, poly(alkylene glycol adipate)s, poly(ethylene glycol adipate), poly(propylene glycol adipate) and poly(butylene glycol adipate), polyvinyl acetate and blends and copolymers thereof.

4. The extended release tablet of claim 3, wherein the pharmaceutically acceptable polymer which is substantially water insoluble is ethyl cellulose.

5. The extended release tablet of any one of claims 1 to 3, wherein the inlay core is in the form of a tablet or compressed slug.

6. The extended release tablet of any one of claims 1 to 3, wherein the inlay core and/or the outer layer independently further comprise at least one other pharmaceutical excipient.

7. The extended release tablet of claim 6, wherein the at least one other pharmaceutical excipient is selected from the group consisting of pharmaceutically acceptable fillers, diluents, pH modifiers, glidants, lubricants, binders, dyes and flavoring agents.

8. The extended release tablet of any one of claims 1 to 7, wherein
the inlay core comprises Paliperidone in about 1-3 % w/w, a filler in about 2-5% w/w, pH modifier in about 5-15% w/w, release modifying polymer in about 5-20% w/w, lubricant in about 0-1 % w/w and glidant in about 0-1 % w/w; and
the outer layer comprises a release modifying polymer in about 50-90% w/w, dye in about 0-1% w/w and lubricant in about 0-1% w/w.

9. The extended release tablet of claim 8, wherein
the inlay core comprises Paliperidone in about 1-3 % w/w, STARLAC in about 2-5% w/w, magnesium carbonate USP in about 5-15% w/w, poly(ethylene oxide) in about 5-20% w/w, stearic acid NF in about 0-1% w/w and silicone dioxide NF in about 0-1 % w/w; and
the outer layer comprises ethyl cellulose in about 50-90% w/w, Ferric Oxide Yellow NF in about 0-1% w/w and stearic acid NF in about 0-1% w/w.

10. A process of making the extended release tablet of any one of claims 1 to 9, comprising
(1) mixing Paliperidone and at least one polymer capable of delaying the release of Paliperidone and capable of swelling upon hydration; and
(2) partially covering the mixture of step (1) with an outer layer comprising a pharmaceutical excipient which is substantially water insoluble to obtain the extended release tablet in the form of an inlay tablet.

11. The process of claim 10, wherein the mixture of step (1) is compressed into a slug or tablet before step (2).

12. The process of claim 10, wherein the mixture of step (1) is compressed into a slug or tablet before step (2); the slug or tablet is mixed with the at least one polymer capable of delaying the release ofPaliperidone and capable of swelling upon hydration to form a mixture; and the mixture is compressed into a tablet before step (2).

13. The process of any one of claims 10 to 12, wherein a filler, pH modifier, glidant and/or lubricant is added in step (1).

14. The process of claim 11 or 12, wherein the slug or tablet formed by compression is milled before being mixed with the at least one polymer capable of delaying the release of Paliperidone and capable of swelling upon hydration to form the mixture to be compressed into a tablet before step (2).

15. The process of any one of claims 10 to 14, wherein the product of step (2) is compressed to obtain the inlay tablet.

## Patentansprüche

1. Paliperidontablette mit verzögerter Freisetzung in der Form einer Tablette mit Einlage, welche umfasst:
(a) einen Einlagekern, der nicht-beschichtetes Paliperidon und wenigstens ein Polymer umfasst, welches die Freisetzung von Paliperidon aus dem Einlagekern verzögern und bei Flüssigkeitszufuhr quellen kann, und
(b) eine äußere Schicht, die einen pharmazeutischen Hilfsstoff umfasst, der im Wesentlichen wasserunlöslich ist, wobei die äußere Schicht teilweise den Einlagekern umschließt.

2. Tablette mit verzögerter Freisetzung nach Anspruch 1, wobei das wenigstens eine Polymer des Einlagelerns ausgewählt ist aus der Gruppe, bestehend aus Polyvinylpyrrolidon, Poly(ethylenoxid), Polysacchariden, hydrophilen Cellulosederivaten, Methylcellulose, Ethylcellulose, Hydroxypropylmethylcellulose, Hydroxyethylcellulose, Hydroxypropylcellulose, Hydroxyethylmethylcellulose, Carboxymethylcellulose und Natrium-Carboxymethylcellulose, bevlrzugt ist das wenigstens eine Polymer des Einlagekerns ausgewählt aus der Gruppe, bestehend aus Methylcellulose, Ethylcellulose, Hydroxypropylmethylcellulose, Hydroxyethylcellulose, Hydroxypropylcellulose, Hydroxyethylmethylcellulose, Carboxymethylcellulose und Natrium-Carboxymethylcellulose, mehr bevorzugt ist das wenigstens eine Polymer des Einlagekerns ausgewählt aus der Gruppe, bestehend aus Poly(ethylenoxid) und Hydroxypropylmethylcellulose.

3. Tablette mit verzögerter Freisetzung nach Anspruch 1 oder Anspruch 2, wobei der pharmazeutische Hilfsstoff, der im Wesentlichen aus der äußeren Schicht wasserunlöslich ist, ein pharmazeutisch verträgliches Polymer ist, das im Wesentlichen wasserunlöslich ist, bevorzugt ist das pharmazeutisch verträgliche Polymer, das im Wesentlichen wasserunlöslich ist, ausgewählt aus der Gruppe, bestehend aus kationischen Copolymeren aus Ethylacrylat und Methylacrylat mit quartären Ammoniumgruppen, Ethylacrylat-Methylmethacrylat-Copolymer mit neutralen Estergruppen, Celluloseestern, Celluloseethern und Celluloseesterethern, Ethylcellulose wie Celluloseacetat, Cellulosediacetat, Cellulosetriacetat und Polyesterpolymeren, Poly(ε-caprolacton)en, Poly(alkylenglycoladipat)en, Poly(ethylenglycoladipat), Poly(propylenglycoladipat) und Poly(butylenglycoladipat), Polyvinylacetat und Gemischen und Copolymeren davon.

4. Tablette mit verzögerter Freisetzung nach Anspruch 3, wobei das pharmazeutisch verträgliche Polymer, das im Wesentlichen wasserunlöslich ist, Ethylcellulose ist.

5. Tablette mit verzögerter Freisetzung nach einem der Ansprüche 1 bis 3, wobei der Einlagekern in der Form einer Tablette oder eines zusammengepressten Blöckchens vorliegt.

6. Tablette mit verzögerter Freisetzung nach einem der Ansprüche 1 bis 3, wobei der Einlagekern und/oder die äußere Schicht unabhängig ferner wenigstens einen anderen pharmazeutischen Hilfsstoff umfasst.

7. Tablette mit verzögerter Freisetzung nach Anspruch 6, wobei der wenigstens eine andere pharmazeutische Hilfsstoff ausgewählt ist aus der Gruppe, bestehend aus pharmazeutisch verträglichen Füllstoffen, Verdünnungsmitteln, pH-Modifikatoren, Fließregulierungsmitteln, Gleitmitteln, Bindemittein, Färbemitteln und Geschmacksstoffen.

8. Tablette mit verzögerter Freisetzung nach einem der Ansprüche 1 bis 7, wobei
der Einlagekern Paliperidon zu etwa 1-3 Gew.-%, einen Füllstoff zu etwa 2-5 Gew.%, einen pH-Modifikator zu etwa 5-15 Gew.-%, ein die Freisetzung modifizierendes Polymer zu etwa 5-20 Gew.-%, ein Gleitmittel zu etwa 0-1 Gew.-% und ein Fließregulierungsmittel zu etwa 0-1 Gew.-% umfasst, und
die äußere Schicht ein die Freisetzung modifizierendes Polymer zu etwa 50-90 Gew.-
%, ein Färbemittel zu etwa 0-1 Gew.-% und ein Gleitmittel zu etwa 0-1 Gew.-% umfasst.

9. Tablette mit verzögerter Freisetzung nach Anspruch 8, wobei
der Einlagekern Paliperidon zu etwa 1-3 Gew.-%, STARLAC zu etwa 2-5 Gew.-%,
Magnesiumcarbonat USP zu etwa 5-15 Gew.-%, Poly(ethylenoxid) zu etwa 5-20 Gew.-%, Stearinsäure NF zu etwa 0-1 Gew.-% und Siliziumdioxid NF zu etwa 0-1 Gew.-% umfasst, und
die äußere Schicht Ethylcellulose zu etwa 50-90 Gew.-%, Eisenoxidgelb NF zu etwa 0-1 Gew.-% und Stearinsäure NF zu wenigstens 0-1 Gew.-% umfasst.

10. Verfahren zur Herstellung einer Tablette mit verzögerter Freisetzung nach einem der Ansprüche 1 bis 9, welches Folgendes umfasst:
(1) Mischen von Paliperidon und wenigstens einem Polymer, das die Freisetzung von Paliperidon verzögern und bei Flüssigkeitszufuhr quellen kann, und
(2) teilweises Bedecken des Gemischs von Stufe (1) mit einer äußeren Schicht, die einen pharmazeutischen Hilfsstoff umfasst, der im Wesentlichen wasserunlöslich ist, um die Tablette mit verzögerter Freisetzung in der Form einer Tablette mit Einlage zu erhalten.

11. Verfahren nach Anspruch 10, wobei das Gemisch von Stufe (1) vor Stufe (2) in ein Blöckchen oder eine Tablette gepresst wird.

12. Verfahren gemäß Anspruch 10, wobei das Gemisch von Stufe (1) vor Stufe (2) in ein Blöckchen oder eine Tablette gepresst wird, das Blöckchen oder die Tablette mit dem wenigstens eine Polymer gemischt wird, das die Freisetzung von Paliperidon verzögern und bei Flüssigkeitszufuhr quellen kann, um ein Gemisch zu bilden, und das Gemisch vor Stufe (2) in eine Tablette gepresst wird.

13. Verfahren nach einem der Ansprüche 10 bis 12, wobei ein Füllstoff, ein pH-Modifikator, ein Fließregulierungsmittel und/oder ein Gleitmittel in Stufe (1) zugefügt wird.

14. Verfahren nach Anspruch 11 oder 12, wobei das/die durch Zusammenpressen geformte Blöckchen oder Tablette gemahlen wird, bevor es/sie mit dem wenigstens einen Polymer, das die Freisetzung von Paliperidon verzögern und bei Flüssigkeitszufuhr quellen kann, gemischt wird, um ein Gemisch zu bilden, das vor Stufe (2) in eine Tablette gepresst wird.

15. Verfahren nach einem der Ansprüche 10 bis 14, wobei das Produkt von Stufe (2) gepresst wird, um eine Tablette mit Einlage zu erhalten.

## Revendications

1. Comprimé de palipéridone à libération prolongée sous la forme d'un comprimé incrusté comprenant :
(a) un noyau incrusté comprenant de la palipéridone non enrobée et au moins un polymère capable de retarder la libération de la palipéridone à partir du noyau incrusté et capable de gonflage lors d'une hydratation ; et
(b) une couche externe comprenant un excipient pharmaceutique qui est sensiblement insoluble dans l'eau, où la couche externe entoure partiellement le noyau incrusté.

2. Comprimé à libération prolongée selon la revendication 1, dans lequel le au moins un polymère du noyau incrusté est choisi dans le groupe consistant en la poly(vinylpyrrolidone), le poly(oxyde d'éthylène), les polysaccharides, les dérivés de cellulose hydrophiles, la méthylcellulose, l'éthylcellulose, l'hydroxypropylméthylcellulose, l'hydroxyéthylcellulose, l'hydroxypropylcellulose, l'hydroxyéthylméthylcellulose, la carboxyméthylcellulose et la carboxyméthylcellulose de sodium, de préférence le au moins un polymère du noyau incrusté est choisi dans le groupe consistant en la méthylcellulose, l'éthylcellulose, l'hydroxypropylméthylcellulose, l'hydroxyéthylcellulose, l'hydroxypropylcellulose, l'hydroxyéthylméthylcellulose, la carboxyméthylcellulose et la carboxyméthylcellulose de sodium, de manière davantage préférée le au moins un polymère du noyau incrusté est choisi dans le groupe consistant en le poly(oxyde d'éthylène) et l'hydroxypropylméthylcellulose.

3. Comprimé à libération prolongée selon la revendication 1 ou la revendication 2, dans lequel l'excipient pharmaceutique qui est sensiblement insoluble dans l'eau de la couche externe est un polymère pharmaceutiquement acceptable qui est sensiblement insoluble dans l'eau, de préférence ledit polymère pharmaceutiquement acceptable qui est sensiblement insoluble dans l'eau est choisi dans le groupe consistant en les copolymères cationiques d'acrylate d'éthyle et d'acrylate de méthyle avec des groupes ammonium quaternaire, un copolymère d'acrylate d'éthyle-méthacrylate de méthyle avec des groupes ester neutres, les esters de cellulose, les éthers de cellulose et les ester éthers de cellulose, l'éthylcellulose telle que l'acétate de cellulose, le diacétate de cellulose, le triacétate de cellulose et les polymères poly(ester), les poly(ε-caprolactones), les poly(adipates d'alkylène glycol), le poly(adipate d'éthylène glycol), le poly(adipate de propylène glycol) et le poly(adipate de butylène glycol), le poly(acétate de vinyle) et leurs mélanges et copolymères.

4. Comprimé à libération prolongée selon la revendication 3, dans lequel le polymère pharmaceutiquement acceptable qui est sensiblement insoluble dans l'eau est l'éthylcellulose.

5. Comprimé à libération prolongée selon l'une quelconque des revendications 1 à 3, dans lequel le noyau incrusté est sous la forme d'un comprimé ou d'une briquette comprimée.

6. Comprimé à libération prolongée selon l'une quelconque des revendications 1 à 3, dans lequel le noyau incrusté et/ou la couche externe comprennent indépendamment en outre au moins un autre excipient pharmaceutique.

7. Comprimé à libération prolongée selon la revendication 6, dans lequel le au moins un autre excipient pharmaceutique est choisi dans le groupe consistant en les charges, diluants, modificateurs de pH, glissants, lubrifiants, liants, colorants et agents aromatisants pharmaceutiquement acceptables.

8. Comprimé à libération prolongée selon l'une quelconque des revendications 1 à 7, dans lequel
le noyau incrusté comprend de la palipéridone à environ 1 à 3 % p/p, une charge à environ 2 à 5 % p/p, un modificateur de pH à environ 5 à 15 % p/p, un polymère modifiant la libération à environ 5 à 20 % p/p, un lubrifiant à environ 0 à 1 % p/p et un glissant à environ 0 à 1 % p/p ; et
la couche externe comprend un polymère modifiant la libération à environ 50 à 90 % p/p, un colorant à environ 0 à 1 % p/p et un lubrifiant à environ 0 à 1 % p/p.

9. Comprimé à libération prolongée selon la revendication 8, dans lequel le noyau incrusté comprend de la palipéridone à environ 1 à 3 % p/p, du STARLAC à environ 2 à 5 % p/p, du carbonate de magnésium USP à environ 5 à 15 % p/p, du poly(oxyde d'éthylène) à environ 5 à 20 % p/p, de l'acide stéarique NF à environ 0 à 1 % p/p et du dioxyde de silicium NF à environ 0 à 1 % p/p ; et
la couche externe comprend de l'éthylcellulose à environ 50 à 90 % p/p, de l'oxyde ferrique jaune NF à environ 0 à 1 % p/p et de l'acide stéarique NF à environ 0 à 1 % p/p.

10. Procédé de fabrication du comprimé à libération prolongée de l'une quelconque des revendications 1 à 9, comprenant les étapes consistant à
(1) mélanger de la palipéridone et au moins un polymère capable de retarder la libération de la palipéridone et capable de gonflage lors d'une hydratation ; et
(2) couvrir partiellement le mélange de l'étape (1) avec une couche externe comprenant un excipient pharmaceutique qui est sensiblement insoluble dans l'eau pour obtenir le comprimé à libération prolongée sous la forme d'un comprimé incrusté.

11. Procédé selon la revendication 10, dans lequel le mélange de l'étape (1) est comprimé en une briquette ou un comprimé avant l'étape (2).

12. Procédé selon la revendication 10, dans lequel le mélange de l'étape (1) est comprimé en une briquette ou un comprimé avant l'étape (2) ; la briquette ou le comprimé est mélangé avec au moins un polymère capable de retarder la libération de la palipéridone et capable de gonflage lors d'une hydratation pour former un mélange ; et le mélange est comprimé en un comprimé avant l'étape (2).

13. Procédé selon l'une quelconque des revendications 10 à 12, dans lequel une charge, un modificateur de pH, un glissant et/ou un lubrifiant sont ajoutés à l'étape (1).

14. Procédé selon la revendication 11 ou 12, dans lequel la briquette ou le comprimé formé par compression est broyé avant d'être mélangé avec le au moins un polymère capable de retarder la libération de la palipéridone et capable de gonflage lors d'une hydratation pour former le mélange à comprimer en un comprimé avec l'étape (2).

15. Procédé selon l'une quelconque des revendications 10 à 14, dans lequel le produit de l'étape (2) est comprimé pour obtenir le comprimé incrusté.
